Europäisches Patentamt

European Patent Office

Office européen des brevets

(11) Veröffentlichungsnummer: **0 369 130**
**A1**

# EUROPÄISCHE PATENTANMELDUNG

(21) Anmeldenummer: **89116739.7**

(22) Anmeldetag: **09.09.89**

(51) Int. Cl.⁵: **C07C 15/16, C07C 7/10**

(30) Priorität: **04.11.88 DE 3837450**

(43) Veröffentlichungstag der Anmeldung:
**23.05.90 Patentblatt 90/21**

(84) Benannte Vertragsstaaten:
**BE DE FR GB IT NL**

(71) Anmelder: **HÜLS AKTIENGESELLSCHAFT**
**Patentabteilung / PB 15 - Postfach 13 20**
**D-4370 Marl 1(DE)**

(72) Erfinder: **Kaufhold, Manfred, Dr.**
**Jasminweg 20**
**D-4370 Marl(DE)**

(54) **Verfahren zur Herstellung von 1.1-Diphenylethan mit guter Geruchsqualität.**

(57) Es war Aufgabe der Erfindung, ein wirtschaftliches Verfahren zur Herstellung von 1.1-Diphenylethan mit guter Geruchsqualität auf Basis des Destillationsrückstandes der Ethylbenzolproduktion zu finden. Die bisher bekannten Verfahren, z. B. Umsetzung von Styrol mit Benzol oder Umsetzung von Benzylchlorid mit Toluol oder die oxidative Kupplung zwischen Ethylbenzol, liefern nur geringe Ausbeuten und benötigen teure Einsatzstoffe.

Gelöst wurde die Aufgabe dadurch, daß man den Destillationsrückstand der Ethylbenzolproduktion zunächst einer Destillation unterwirft, das Destillat dann mit Schwefelsäure behandelt, die Schwefelsäure-Phase abtrennt, die Ölphase mit Natronlauge und Wasser wäscht und die so erhaltene Ölphase einer zweiten Destillation unterwirft.

Das Produkt wird auf dem Riechstoffsektor angewendet.

EP 0 369 130 A1

EP 0 369 130 A1

## Verfahren zur Herstellung von 1.1-Diphenylethan mit guter Geruchsqualität

1.1-Diphenylethan - auch als Methyldiphenylmethan bezeichnet - ist ein bekannter, aber bisher wirtschaftlich wenig bedeutender Riechstoff. Er wurde von Steffen Arctander beschrieben, siehe S. Arctander, Perfume and Flavor Chemicals, published by the Author 1969, Montclair, N.J. (USA), Nr. 2007.

Eine Synthese dieses Riechstoffs basiert auf der Friedel-Crafts-Reaktion von Styrol mit Benzol, siehe Spilker u. Schade, Chem. Berichte 65 (1932), 1686. Sie erhielten 1.1-Diphenylethan in nur 25 %iger Ausbeute. Auch andere, in der Literatur beschriebene Verfahren, wie z. B. die Reaktion von Benzylchlorid mit Toluol und Kupferchlorid als Katalysatoren (US-PS 3 679 760) oder die oxidative Kupplung zwischen Ethylbenzol und Benzol mit einem aus Aluminium- und Kupferchlorid bestehendem Katalysator (US-PS 3 631 211) liefern nur geringe Ausbeuten und/oder benötigen teure Einsatzstoffe, so daß eine technische Verwertung nicht in Frage kommt.

Aus Gründen der Wirtschaftlichkeit sind dagegen Verfahren interessanter, mit denen man 1.1-Diphenylethan aus dem Destillationsrückstand der Ethylbenzolproduktion gewinnen konnte, siehe EP-A 98 677 und C.A. 73 (23): 120212z sowie C.A. 94 (20): 167136b.

Wie aus der EP-A 98 677 hervorgeht, ist auf diese Weise aber kein reines 1.1-Diphenylethan zu gewinnen, da eine Reihe von Verunreinigungen einen sehr ähnlichen Siedepunkt besitzen. Wahrscheinlich ist aus diesem Grund die Gewinnung von 1.1-Diphenylethan mit guter Geruchsqualität aus dem Destillationsrückstand der Ethylbenzolproduktion bisher nicht versucht worden oder bisher nicht gelungen.

Hieraus ergab sich die Aufgabe, ein einfaches und wirtschaftliches Verfahren zur Herstellung von 1.1-Diphenylethan mit guter Geruchsqualität auf Basis des Destillationsrückstandes der Ethylbenzolproduktion zu finden.

Gegenstand der Erfindung ist daher ein Verfahren zur Herstellung von 1.1-Diphenylethan mit guter Geruchsqualität aus dem Destillationsrückstand der Ethylbenzolproduktion, welches dadurch gekennzeichnet ist, daß man den Rückstand zunächst einer ersten Detillation unterwirft, wobei ein 60 bis 90 %iges Produkt erhalten wird, dieses Destillat mit einer konzentrierten 85 bis 95 %igen Schwefelsäure bei Temperaturen von -10 bis +50 °C behandelt, die Schwefelsäurephase abtrennt, die Ölphase mit Natronlauge und Wasser wäscht und anschließend noch eine zweite Destillation durchführt.

Aus olfaktorischer Sicht hat der Destillationsrückstand der Ethylbenzolproduktion einen unangenehmen technischen und an Teer erinnernden Geruch. Wenn man den Rückstand durch aufwendige fraktionierte Destillation reinigt, was in der DE-OS 24 01 373 eingehend beschrieben ist, erhält man Fraktionen mit Gehalten an 1.1-Diphenylethan von ca. 82 bis zu 92 %. Der technische Aufwand ist bei dieser Destillation sehr groß und die Ausbeute an Destillat sehr gering. Die Geruchsqualität aller Fraktionen ist nicht wesentlich besser als die des Ausgangsproduktes und entspricht keinesfalls der eines üblichen Riechstoffs.

Überraschend erhält man erfindungsgemäß 1.1-Diphenylethan mit guter Geruchsqualität auf einfache Weise aus dem Destillationsrückstand der Ethylbenzolproduktion, indem man folgende Reinigungsstufen durchführt:

1. Destillation I
2. Waschen des Destillats mit Schwefelsäure, Natronlauge und Wasser
3. Destillation II

Während wiederholte Destillationen nicht zu Geruchsverbesserungen führen, bewirkt schon eine einfache Wasserwäsche eine starke Verringerung des Teergeruchs. Effektiver ist eine Wäsche mit Natronlauge und die beste Wirkung wird erreicht durch ein Behandeln mit Schwefelsäure und anschließendem Waschen mit Natronlauge und Wasser.

Zur weiteren Qualitätsverbesserung wird in einer 3. Stufe die gewaschene trübe, leicht gelbe Ölphase destilliert. Überraschenderweise fallen bei dieser Destillation II Vorläufe mit unangenehmem Geruch an, die abgetrennt werden. Der dann überdestillierende Hauptlauf besitzt eine gute Geruchsqualität. Der Geruch besitzt kaum die im "Arctander" erwähnte harte, metallische Note, sondern ist weiche und "rund".

Zur praktischen Durchführung ist folgendes wichtig:

Die Destillation I ist eine einfache fraktionierte Destillation, bei der z. B. bei einem Vakuum von 30 mbar in einem Siederbereich von ca. 150 bis 170 °C, vorzugsweise von 153 bis 161 °C, ein Produkt anfällt, das einen Gehalt an 1.1-Diphenylethan von über 60 %, vorzugsweise über 80 %, hat.

Das Destillat wird zunächst bei Temperaturen von -10 °C bis +50 °C, vorzugsweuse bei 0 bis 20 °C, mit einer Schwefelsäure gerührt, deren Gehalt bei 85 bis 95 % liegt, über einen Zeitraum von 10 Minuten bis 5 Stunden, vorzugsweise 1 bis 2 Stunden. Zur Neutralisation wird die Ölphase anschließend mit verdünnter Natronlauge und danach mit Wasser gewaschen. Die trübe, hellgelbe Ölphase wird nochmals destilliert, wobei es nur wichtig ist, einen Vorlauf abzutrennen, da dieser einen unangenehmen Geruch

2

besitzt. Weitere Angaben sind dem Beispiel zu entnehmen.

Es besteht ein großes Interesse an Riechstoffen, die leicht und wirtschaftlich herstellbar sind.

Das erfindungsgemäße einfache Verfahren erfüllt diese Bedingungen und liefert einen an sich bekannten Riechstoff mit überraschend guter Riechstoffqualität, der besonders wegen seiner großen chemischen Beständigkeit geschätzt wird.

Beispiel

Das Reinigungsverfahren besteht aus folgenden Stufen:
1. Destillation I
2. Waschen des Destillats mit Schwefelsäure, Natronlauge und Wasser
3. Destillation II

1. Destillation I

Die Destillation I wird an einer 0,5 m langen, mit Multifil-Füllkörpern gefüllten Glaskolonne durchgeführt. Die Auftrennung der Fraktionen erfolgt einfachheitshalber nach Siedepunkten.

Einsatz zur Destillation 1 000 g:

| Fraktion-Nr. | Temperaturen in °C | | Druck mbar | Gewicht in g | Verhältnis Rücklauf zur Abnahme |
|---|---|---|---|---|---|
| | Kopf | Sumpf | | | |
| 1 | 119-150 | 161-176 | 30 | 164 | 1 : 1 |
| 2 | 150-153 | 176-173 | 30 | 21 | 1 : 1 |
| 3 | 153-158 | 173-185 | 30 | 207 | 1 : 1 |
| 4 | 158 | 185-190 | 30 | 100 | 1 : 1 |
| 5 | 158-161 | 190 | 30 | 37 | 1 : 1 |
| Rückstand | | | | 470 | |
| | | | | 999 | |

Die GC-Analysen der Fraktionen 1 bis 5 zeigen folgende Gehalte an 1.1-Diphenylethan = DPE und an dem etwas höher siedenden 1.2-Diphenylethan = Bibenzyl = BBZ:

| Fraktion-Nr. | DPE | BBZ |
|---|---|---|
| 1 | nicht analysiert, verworfen | |
| 2 | 34,7 | < 0,1 |
| 3 | 83,3 | 0,4 |
| 4 | 92,3 | 1,4 |
| 5 | 82,6 | 5,8 |

Der Rückstand und die Fraktionen 1 und 2 werden verworfen. Da der Gehalt an DPE in den Fraktionen 3 bis 5 über 80 % ist, werden sie zusanmengegeben und weiterverarbeitet. Destillat-Ausbeute: 34 %

2. Wäschen

Die im folgenden beschriebene Reinigung wird mit einem ca. 80 %igen DPE durchgeführt.
Einsatz: 600 ml = 598 g DPE (80 %ig)

50 ml = 86 g $H_2SO_4$ technisch (90 %ig)

DPE wird vorgelegt, unter Rühren auf 10 °C abgekühlt und die Schwefelsäure in 10 Minuten zugetropft. Dann wird 2 Stunden bei 10 °C gerührt. Bei der anschließenden Phasentrennung fallen 88 g Schwefelsäure-Phase mit einem Gehalt von 83,4 % an. Die Ölphase wird mit 50 g Natronlauge (5 %ig) geschüttelt. Die Trennung ergibt 49 g Abwasser mit 2 % Natriumhydroxid. Danach wird die Ölphase mit 100 g Wasser und 4 g Natriumcnlorid geschüttelt.

Die Phasentrennung ergibt:

594 g Ölphase

102 g wäßrige Phase Kohlenstuffgehalt 0,4 g pro l)

## 3. Destillation II

Die feuchte, gelbe, trübe Ölphase wird an einer 0,5 m langen, (nit Multifil-Füllkorpern gefüllten Glaskolonne in folgender Weise fraktioniert:

Einsatz (feucht) 594 g

| Fraktion-Nr. | Temperaturen in °C | | Druck mbar | Gewicht in g | Verhältnis Rücklauf zur Abnahme |
|---|---|---|---|---|---|
| | Kopf | Sumpf | | | |
| 1 | 142-153 | 155-158 | 30 | 37 | 5 : 1 |
| 2 | 153 | 158 | 30 | 28 | 5 : 1 |
| 3 | 153 | 158 | 30 | 495 | 5 : 1 |
| | 154 | 161 | | | 1 : 1 |
| Rückstand | | | | 23 | |
| Kühlfalle | | | | 2 | |
| | | | | 585 | |

Die GC-Analysen der Fraktionen 1 bis 3 zeigen folgende Gehalte an DPE und BBZ:

| Fraktion-Nr. | DPE | BBZ |
|---|---|---|
| 1 | 53,0 | 0,2 |
| 2 | 67,6 | 0,3 |
| 3 | 86,0 | 2,0 |

Die Fraktionen 1 und 2 haben unangenehme Nebengerüche und werden verworfen. Der Hauptlauf, Fraktion 3, hat eine gute Geruchsqualität.

Die Ausbeute an diesem Riechstoff mit einem DPE-Gehalt von über 85 % und gutem Geruch beträgt ca. 30 %, bezogen auf eingesetzten Rückstand der Ethylbenzolproduktion.

## Ansprüche

1. Verfahren zur Herstellung von 1.1-Diphenylethan mit guter Geruchsqualität aus dem Destillationsrückstand der Ethylbenzolproduktion, dadurch gekennzeichnet, daß man den Rückstand zunächst einer ersten Destillation unterwirft, wobei ein 60 bis 90 %iges Produkt erhalten wird, dieses Destillat mit einer konzentrierten 85 bis 95 %igen Schwefelsäure bei Temperaturen von -10 bis +50 °C behandelt, die Schwefelsäure-Phase abtrennt, die Ölphase mit Natronlauge und Wasser wäscht und anschließend noch eine zweite Destillation durchführt.

2. Verfahren nach Anspruch 1,
dadurch gekennzeichnet,
daß man die Behandlung mit Schwefelsäure vorzugsweise bei 0 bis 20 °C durchführt.

3. Verfahren nach Anspruch 1,
dadurch gekennzeichnet,
daß man die Behandlung mit Schwefelsäure 10 Minuten bis 5 Stunden durchführt.

4. Verfahren nach Anspruch 3,
dadurch gekennzeichnet,
daß man die Behandlung mit Schwefelsäure vorzugsweise 1 bis 2 Stunden durchführt.

Europäisches Patentamt

**EUROPÄISCHER RECHERCHENBERICHT**

Nummer der Anmeldung

EP 89 11 6739

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 098 677 (GULF RESEARCH & DEVELOPMENT CO.) <br> * Seite 5, Zeilen 17-35 * <br> --- | 1 | C 07 C 15/16 <br> C 07 C 7/10 |
| Y | CH-A- 373 766 (STAMICARBON) <br> * Seite 1, Zeilen 1-37 * <br> --- | 1 | |
| A | BE-A- 665 125 (METALLGESELLSCHAFT) <br> * Ansprüche 1,2 * <br> --- | 1 | |
| D,A | BERICHTE DER DEUTSCHEN CHEMISCHEN GESELLSCHAFT <br> Jahrgang 65, 1932, Seiten 1686-1689, Verlag Chemie Berlin, D; A. SPILKER et al.: "Ueber Anlagerungsprodukte von Styrol an aromatische Kohlenwasserstoffe" * Seite 1687, Absatz 5 - Seite 1688, Absatz 1 * <br> --- | 1 | |
| A,P | CHEMICAL ABSTRACTS <br> Band 110, Nr. 11, 22. Mai 1989, Zusammenfassung Nr. 192424a; & SU - A - 1 425 184 (G.M. SAKHNAZARYAN) 23.09.1988 <br> --- | 1 | **RECHERCHIERTE SACHGEBIETE (Int. Cl.5)** <br><br> C 07 C 15/00 <br> C 07 C 7/00 |
| A | DE-A-3 227 491 (ALLIED CORP.) <br> * Anspruch 1 * <br> ----- | 1 | |

Der vorliegende Recherchenbericht wurde für alle Patentansprüche erstellt

| Recherchenort | Abschlußdatum der Recherche | Prüfer |
|---|---|---|
| BERLIN | 26-01-1990 | PROBERT C.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)